# EUROPÄISCHE PATENTSCHRIFT

(11) **EP 1 035 878 B1**
(45) Veröffentlichungstag und Bekanntmachung des Hinweises auf die Patenterteilung: **10.10.2001**
(21) Anmeldenummer: 98964460.4
(22) Anmeldetag: 30.11.1998
(51) Int. Cl.: A61L 27/00, A61F 2/32

(54) **KÜNSTLICHES GELENK EINER PROTHESE**
ARTIFICIAL JOINT OF A PROSTHESIS
ARTICULATION ARTIFICELLE D'UNE PROTHESE

(30) Priorität: 28.11.1997 DE 19752674
(43) Veröffentlichungstag der Anmeldung: 20.09.2000
(73) Patentinhaber: Ceramtec AG Innovative Ceramic Engineering, 73207 Plochingen (DE)
(72) Erfinder: DEPPISCH, Werner, verstorben (DE); PFAFF, Hans-Georg, D-73760 Ostfildern (DE); WILLMANN, Gerd, D-70771 Leinfelden (DE)
(74) Vertreter: Uppena, Franz, Dr.
(86) Internationale Anmeldenummer: EP9807709
(87) Internationale Veröffentlichungsnummer: WO9927871

(56) Entgegenhaltungen:
- DE-A- 2 540 077
- US-A- 4 950 294
- WILLMANN G: "20 JAHRE ALUMINIUMOXIDKERAMIK F}R DIE MEDIZINTECHNIK. ALUMINA CERAMIC LOOKS BACK ON 20 YEARS OF USE IN MEDICAL APPLICATIONS" BIOMEDIZINISCHE TECHNIK, Bd. 39, Nr. 4, 1. April 1994, Seiten 73-78, XP000439856

## Beschreibung

Die Erfindung betrifft einen keramischen Sinterwerkstoff für Prothesen oder Implantate sowie ein künstliches Gelenk einer Prothese, bei dem mindestens ein Gelenkpartner aus diesem keramischen Werkstoff besteht.

Bei Prothesen mit künstlichen Gelenken können aufgrund der erforderlichen Verträglichkeit mit dem menschlichen oder auch tierischen Gewebe, der sogenannten Biokompatibilität, und der hohen Reibbelastung der Gelenkpartner nur wenige Werkstoffe eingesetzt werden. Von den metallischen Werkstoffen haben sich Legierungen auf der Basis von Titan, beispielsweise TiAl6V4, TiAl6Nb7, und Kobalt-Chrom (CoCrMo) bewährt. Von den Kunststoffen kommt insbesondere Polyethylen bei den Pfannen der Hüftgelenk-Endoprothesen zum Einsatz und Aluminiumoxid und Zirkonoxid sind die brauchbaren Keramikwerkstoffe. Während Prothesen aus einem der genannten Werkstoffe allein im menschlichen oder tierischen Gewebe keine Probleme aufwerfen, können die Werkstoffe untereinander nicht beliebig miteinander kombiniert werden, wenn sich dadurch Reibpaarungen ergeben, wie es in künstlichen Gelenken der Fall ist. Zu beachten ist außerdem die Anwesenheit der Körperflüssigkeit als korrosives Medium.

Besonders bewährt haben sich in Gelenken Paarungen von Gelenkpartnern aus Aluminiumoxid, wie es beispielsweise in Hüftgelenk-Endoprothesen der Fall ist. Sowohl die Kugel als auch die Pfanne sind aus dem selben Keramikwerkstoff gefertigt. Wie es aus der Veröffentlichung "Wear characteristics of ceramic-on-ceramic for hip endoprostheses" von Früh, Willmann und Pfaff aus "Biomaterials", 18 (1997), No. 12, Seiten 873 bis 876, bekannt ist, können Gelenkpartner aus Aluminiumoxid und aus Zirkonoxid nicht miteinander gepaart werden, weil dadurch ein nichttolerierbarer Verschleiß auftritt. Auch eine Paarung von Gelenkpartnern aus Zirkonoxid ist aufgrund des Verschleißes nicht möglich.

Mit Zirkonoxid-Keramiken lassen sich allerdings Werkstücke herstellen, die bei vergleichbaren Abmessungen eine höhere Sicherheit insbesondere gegen höhere Wechselbelastungen aufweisen. Aus diesem Grund ist es zum Beispiel nicht sinnvoll, die Kugelkopfdurchmesser von Hüftgelenk-Endoprothesen unterhalb von 28 mm Durchmesser aus Aluminiumoxid herzustellen. Durchmesser unter 28 mm sind aber problemlos aus Keramiken aus Zirkonoxid mit der geforderten Festigkeit und Dauerwechselfestigkeit realisierbar. Der Nachteil besteht allerdings darin, daß Kugeln aus Zikronoxid-Keramik nicht gegen Pfannen aus Aluminiumoxid und Zirkonoxid gelagert werden können. Es ist deshalb üblich, Kugeln aus Zirkonoxid in Pfannen von Polyethylen zu lagern, was jedoch Partikelabrieb von Polyethylen verursacht und dadurch medizinische Probleme zur Folge hat.

Aus der US-PS 4,950,294 ist ein Werkstoff für künstliche Knochen oder Implantate bekannt, der hauptsächlich aus Zirkonoxid mit einem Anteil aus Aluminiumoxid besteht. Dabei können die Anteile der beiden Werkstoffe für den jeweiligen Verwendungszweck optimal aufeinander abgestimmt werden. Aus dieser Veröffentlichung ist es nicht bekannt, Komponenten aus den benannten Werkstoffen als Gelenkpartner zu einem verschleißbehafteten Gelenk zusammenzustellen.

In der Veröffentlichung von G. Willmann, 20 Jahre Aluminiumoxidkeramik für die Medizintechnik, Biomed. Technik, 39 (1994), 73 - 78, werden die Eigenschaften der bekannten Werkstoffe künstlicher Hüftgelenkendoprothesen vorgestellt. Dabei werden die möglichen Kombinationen von Pfannen und Kugelköpfen aus den für eine Implantation geeigneten Werkstoffen bewertet. Als keramische Sinterwerkstoffe sind Aluminiumoxid und Zirkonoxid einzeln angegeben. Die Prothesenkomponenten bestehen demnach jeweils nur aus einem dieser keramischen Werkstoffe. Als nicht geeignet werden Werkstoffpaarungen von Aluminiumoxid mit Zirkonoxid und Zirkonoxid mit Zirkonoxid in künstlichen Hüftgelenken angesehen.

Es ist deshalb die Aufgabe der vorliegenden Erfindung, für Prothesen mit Gelenkpartnern aus keramischen Werkstoffen mögliche Werkstoffpaarungen vorzustellen, wodurch neue konstruktive Gestaltungen mit optimalen Verschleißverhalten ermöglicht werden.

Die Lösung der Aufgabe erfolgt erfindungsgemäß mit Hilfe des ersten Anspruchs. Vorteilhafte Ausgestaltungen der Erfindung werden in den Unteransprüchen beansprucht.

Die erfindungsgemäßen Werkstoffpaarungen ermöglichen künstliche Gelenke von Prothesen, die sich beispielsweise aus Gründen des Verschleißes zuvor nicht verwirklichen ließen.

Der Werkstoff aus Zirkonoxid mit einem Zusatz von 0,1 bis 40 Gewichtsprozent Aluminiumoxid bietet außerdem die Möglichkeit, Abmessungen der Gelenkpartner zu realisieren, beispielsweise den Durchmesser von Kugeldurchmessern der Hüftgelenk-Endoprothesen, die unter 28 mm liegen, wobei die guten thermischen und mechanischen Eigenschaften des Zirkonoxids mit den hervorragenden tribologischen Eigenschaften des Aluminiumoxids kombiniert werden.

Besonders gute tribologische Eigenschaften, Verschleißfestigkeit, in Verbindung mit gutem thermischen und mechanischen Verhalten, insbesondere geringe Erwärmung und hohe Festigkeit bei Dauerwechselbelastung, bietet ein Sinterwerkstoff aus Zirkonoxid mit einem Zusatz von 15 bis 25 Gewichtsprozent Aluminiumoxid.

Eine Stabilisierung der Zirkonoxid-Phasen wird vorteilhaft dadurch erreicht, wenn der Anteil der bekannten Stabilisatoren, beispielsweise Seltene-Erden-Oxide, Erdalkalioxide, Titanoxid, Chromoxid oder Hafniumoxid, unter 10 Gewichtsprozent liegt.

Vorteilhaft ist es weiterhin, wenn beide Gelenkpartner die selbe Stoffzusammensetzung aufweisen. Dadurch sind die Gleitpartner im Abriebverhalten identisch. Mit zunehmendem Anteil von Aluminiumoxid steigt der Widerstand gegen den Verschleiß.

Es kann aber durchaus von Vorteil sein, wenn einer der Gelenkpartner einen höheren Zirkonoxidanteil aufweist als der andere Partner. Das kann beispielsweise bei den Kugeln der Hüftgelenk-Endoprothesen der Fall sein, um die günstigen Werkstoffeigenschaften von Zirkonoxid, insbesondere im Hinblick auf die Kugelabmessungen, vollständig ausnutzen zu können.

Die Werkstoffzusammensetzung macht es auch möglich, daß ein Gelenkpartner aus Zirkonoxid mit einem Aluminiumoxidanteil von über 5 Gewichtsprozent mit einem Gelenkpartner aus reinem Aluminiumoxid gepaart werden kann. Dadurch können die guten tribologischen Eigenschaften des Aluminiumoxids weitestgehend ausgenutzt werden.

Die Eigenschaften der Werkstoffzusammensetzung lassen sich vorteilhaft in einem künstlichen Hüftgelenk ausnutzen, wobei die Kugel des Gelenks aus dem Werkstoff mit dem höheren Zirkonoxid-Anteil besteht. Da insbesondere die Kugel der am stärksten belastete Gelenkpartner ist, werden mit einer solchen Werkstoffzusammensetzung die guten tribologischen Eigenschaften des Aluminiumoxids mit den guten thermischen und mechanischen Eigenschaften des Zirkonoxids kombiniert.

Aufgrund der mechanischen und thermischen Werkstoffeigenschaften des Zirkonoxids ist es vorteilhaft möglich, Kugeln von Hüftgelenkprothesen mit einem Durchmesser kleiner als 28 mm herzustellen und mit einer Pfanne aus einer Aluminiumoxid-Keramik zu paaren, um ein optimales Verschleißverhalten zu ermöglichen.

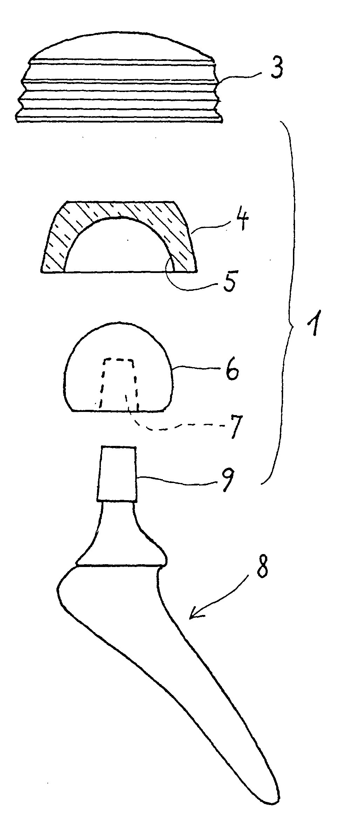

Die Abbildung zeigt schematisch eine modular aufgebaute Hüftgelenk-Endoprothese aus dem erfindungsgemäßen Werkstoff in ihren Einzelteilen.

Die Hüftgelenk-Endoprothese 1 besteht aus einer zweiteiligen Pfanne 2, die mit ihrem metallischen Pfannengehäuse 3 im Knochen verankert ist und einen keramischen Pfanneneinsatz 4 aufnimmt, in dessen hohlkugelförmigen Lagerfläche 5 sich die Kugel 6 bewegen kann. Die Kugel 6 besteht ebenfalls aus Keramik und weist eine konische Bohrung 7 auf. Der Schaft 8 aus einem bionierten Metall, beispielsweise aus der Kobaltlegierung CoCrMo, weist einen Zapfen 9 auf, dessen Konizität mit der Konizität der Bohrung 7 in der Kugel 6 übereinstimmt. Der Zapfen 9 wird durch Preßsitz in der Bohrung 7 der Kugel 6 verankert.

Im vorliegenden Ausführungsbeispiel ist von den Gelenkpartnern, der Kugel 6 und dem Pfanneneinsatz 4, die Kugel 6 aus Zirkonoxid mit einem Aluminiumoxid-Anteil von über 5 Gewichtsprozent. Der Pfanneneinsatz 4 besteht aus einer Aluminiumoxidkeramik in der bekannten Zusammensetzung. Die Kugel 6 besitzt einen Durchmesser, der kleiner als 28 mm ist. Die erfindungsgemäße Werkstoffzusammensetzung ermöglicht einen wesentlich kleineren Kugeldurchmesser, als er mit einem Werkstoff aus reinem Aluminiumoxid möglich ist. Eine Kugel aus dem angegebenen Werkstoff läßt sich gefahrlos mit einem Pfanneneinsatz aus Aluminiumoxid zu einer Gelenkpaarung zusammensetzen, ohne daß abrasive und korrosive Schäden auftreten.

## Patentansprüche

1. Künstliches Gelenk einer Prothese, bei dem die Gelenkpartner aus keramischen Sinterwerkstoffen bestehen, **dadurch gekennzeichnet, daß** die Sinterwerkstoffe im wesentlichen Aluminiumoxid und Zirkonoxid sind und daß mindestens einer (6) der Gelenkpartner (4, 6) aus einem Sinterwerkstoff aus Zirkonoxid mit einem Zusatz von 0,1 bis 40 Gewichtsprozent Aluminiumoxid besteht.

2. Künstliches Gelenk nach Anspruch 1, **dadurch gekennzeichnet, daß** der Sinterwerkstoff aus Zirkonoxid mit einem Zusatz von vorzugsweise 15 bis 25 Gewichtsprozent Aluminiumoxid besteht.

3. Künstliches Gelenk nach Anspruch 1 oder 2, **dadurch gekennzeichnet, daß** bei einem Gelenkpartner (6) mit einem Werkstoff aus Zirkonoxid und Aluminiumoxid der Werkstoff einen bekannten Stabilisator für die Zirkonoxid-Phase in einem Anteil von unter 10 Gewichtsprozent aufweist.

4. Künstliches Gelenk nach einem der Ansprüche 1 bis 3, **dadurch gekennzeichnet, daß** beide Gelenkpartner die selbe Werkstoffzusammensetzung aufweisen.

5. Künstliches Gelenk nach einem der Ansprüche 1 bis 3, **dadurch gekennzeichnet, daß** der Werkstoff des einen Gelenkpartners einen höheren Zirkonoxidanteil aufweist als der Werkstoff des anderen Gelenkpartners.

6. Künstliches Gelenk nach einem der Ansprüche 1 bis 3, **dadurch gekennzeichnet, daß** einem Gelenkpartner (6) aus Zirkonoxid mit einem Aluminiumoxid-Anteil von über 5 Gewichtsprozent ein Gelenkpartner (4) aus Aluminiumoxid zugeordnet ist.

7. Künstliches Gelenk nach einem der Ansprüche 1 bis 6, **dadurch gekennzeichnet, daß** das Gelenk ein künstliches Hüftgelenk (1) ist.

8. Künstliches Gelenk nach Anspruch 7, **dadurch gekennzeichnet, daß** der Werkstoff der Kugel (6) des Gelenks (1) einen höheren Zirkonoxid-Anteil aufweist als der Werkstoff des keramischen Pfanneneinsatzes (4).

9. Künstliches Gelenk nach Anspruch 7, **dadurch gekennzeichnet, daß** ausschließlich der Werkstoff der Kugel (6) des Gelenks (1) einen Zirkonoxid-Anteil aufweist.

10. Künstliches Gelenk nach Anspruch 8 oder 9, **dadurch gekennzeichnet, daß** der Durchmesser der Kugel (6) des Gelenks (1) kleiner als 28 mm ist.

## Claims

1. Artificial joint of a prosthesis, in which the joint partners consist of ceramic sintered materials, **characterised in that** the sintered materials are substantially aluminium oxide and zirconium oxide and **in that** at least one (6) of the joint partners (4, 6) consists of a sintered material of zirconium oxide with an addition of 0.1 to 40 % by weight aluminium oxide.

2. Artificial joint according to claim 1, **characterised in that** the sintered material consists of zirconium oxide with an addition of preferably 15 to 25% by weight aluminium oxide.

3. Artificial joint according to claim 1 or 2, **characterised in that**, in the case of a joint partner (6) having a zirconium oxide and aluminium oxide material, the material has a known stabilizer for the zirconium-oxide phase in a proportion of less than 10 % by weight.

4. Artificial joint according to one of claims 1 to 3, **characterised in that** both joint partners have the same material composition.

5. Artificial joint according to one of claims 1 to 3, **characterised in that** the material of the one joint partner has a higher proportion of zirconium oxide than the material of the other joint partner.

6. Artificial joint according to one of claims 1 to 3, **characterised in that** a joint partner (4) of aluminium oxide is associated with a joint partner (6) of zirconium oxide having a proportion of aluminium oxide of more than 5 % by weight.

7. Artificial joint according to one of claims 1 to 6, **characterised in that** the joint is an artificial hip joint (1).

8. Artificial joint according to claim 7, **characterised in that** the material of the ball (6) of the joint (1) has a higher proportion of zirconium oxide than the material of the ceramic socket insert (4).

9. Artificial joint according to claim 7, **characterized in that** exclusively the material of the ball (6) of the joint (1) has a zirconium-oxide content.

10. Artificial joint according to claim 8 or 9, **characterised in that** the diameter of the ball (6) of the joint (1) is smaller than 28 mm.

## Revendications

1. Articulation artificielle dans une prothèse, dans laquelle les éléments de l'articulation sont formés de matériaux frittés céramiques, **caractérisée en ce que** les matériaux frittés sont essentiellement de l'oxyde d'aluminium et de l'oxyde de zirconium et **en ce qu'**au moins l'un (6) des éléments (4, 6) de l'articulation est formé d'un matériau fritté à base d'oxyde de zirconium auquel on a ajouté de 0,1 à 40 % en poids d'oxyde d'aluminium.

2. Articulation artificielle selon la revendication 1, **caractérisée en ce que** le matériau fritté est formé d'oxyde de zirconium auquel on a ajouté de 15 à 25 % en poids d'oxyde d'aluminium.

3. Articulation artificielle selon la revendication 1 ou 2, **caractérisée en ce que**, lorsqu'un élément d'articulation (6) est réalisé en un matériau à base d'oxyde de zirconium et d'oxyde d'aluminium, le matériau contient un stabilisateur connu pour l'oxyde de zirconium, dans une proportion inférieure à 10 % en poids.

4. Articulation artificielle selon une des revendications 1 à 3, **caractérisée en ce que** les deux éléments de l'articulation présentent la même composition de matériau.

5. Articulation artificielle selon une des revendications 1 à 3, **caractérisée en ce que** le matériau de l'un des deux éléments de l'articulation présente une proportion d'oxyde de zirconium plus élevée que le matériau de l'autre élément de l'articulation.

6. Articulation artificielle selon une des revendications 1 à 3, **caractérisée en ce qu'**à un élément (6) de l'articulation réalisé en oxyde de zirconium avec une quantité d'oxyde d'aluminium supérieure à 5% en poids est associé un élément d'articulation (4) en oxyde d'aluminium.

7. Articulation artificielle selon une des revendications 1 à 6, **caractérisée en ce que** l'articulation est une articulation artificielle de hanche (1).

8. Articulation artificielle selon la revendication 7, **caractérisée en ce que** le matériau de la rotule (6) de l'articulation (1) contient une quantité d'oxyde de zirconium plus élevée que le matériau de la cuvette (4) en céramique.

9. Articulation artificielle selon la revendication 7, **caractérisée en ce que** seul le matériau de la rotule (6) de l'articulation (1) contient de l'oxyde de zirconium.

10. Articulation artificielle selon la revendication 8 ou 9, **caractérisée en ce que** le diamètre de la rotule (6) de l'articulation (1) est inférieur à 28 mm.
